# EUROPEAN PATENT APPLICATION

(11) **EP 4 732 758 A1**
(43) Date of publication of application: **29.04.2026**
(21) Application number: 24208867.2
(22) Date of filing: 25.10.2024
(51) Int. Cl.: A61B 5/00

(54) **METHOD AND SYSTEM FOR DETECTION OF TOOTH CRACKS IN DIGITAL 3D MODELS OF TEETH**

(71) Applicant: 3Shape A/S, 1060 Copenhagen K (DK)
(72) Inventor: VANNAHME, Christoph, 1060 Copenhagen K (DK); PEDERSEN, Troels Holm, 1060 Copenhagen (DK); MADHAN, Sivaranjani, 1060 Copenhagen K (DK); MICHOU, Stavroula, 1060 Copenhagen K (DK)
(74) Representative: Zacco Denmark A/S

(57) **Abstract**

A computer-implemented method for detecting tooth cracks on a digital 3D model (501) of a dental situation is disclosed. The method comprises receiving the digital 3D model (501) of the dental situation, generating an input (301, 401) for a trained neural network (300, 400) for tooth crack detection, wherein the input (301, 401) comprises at least a part of the digital 3D model (501) with associated color information. The method further comprises detecting a tooth crack (100) on the at least part of the digital 3D model (501) by applying the trained neural network (300, 400) to the generated input (301, 401). Further, the method comprises determining a severity level of the detected tooth crack (100) based on fluorescence information associated with the at least part of the digital 3D model (501) and displaying the at least part of the digital 3D model (501), the detected tooth crack (100) and the severity level of the detected tooth crack (100).

## Description

### Technical field

The disclosure relates to a computer-implemented method and system for detecting tooth cracks on a digital 3D dental model of a patient's dentition.

### Background

Cracked tooth syndrome (CTS) is among the most prevalent dental diseases of today. Even though it is most prevalent with elderly patients, the number of reported cases of CTS among the younger patients is on the rise.

Tooth cracks may appear due to a number of different causes. Some causes include pressure from teeth grinding, large fillings that weaken the tooth integrity, particular hard food consumption, blows to the mouth, age, change in temperature and gum disease. Symptoms of tooth cracks include erratic pain on mastication, pain on release of biting pressure, thermal or osmotic hyper sensibility or sudden occurrence of pain on an unrestored or only slightly restored tooth.

The major problem with tooth cracks is the potential for bacteria penetration, which could lead to inflammation and disease. The pulp of the affected tooth may be compromised, gingival diseases could appear such as pulpitis, pulp necrosis or apical periodontitis. These conditions may lead to a need for endodontic surgery or even tooth extraction.

However, many teeth with cracks can be saved if the cracks are timely identified and classified.

Tooth cracks are generally difficult to detect. A common approach for clinical diagnosis of tooth cracks is based on dental history or on direct visual inspection of teeth by dental practitioners. Although visual inspection is useful, tooth cracks are not often visible without the aid of magnifying loupes or similar devices. Another method to evaluate suspected cracks is to apply careful periodontal probing to disclose the presence of an isolated periodontal pocket that often indicates the presence of a split tooth. Further, dye staining may be used to highlight fracture lines of teeth. A disadvantage with this method is that it may take up to five days for the method to be effective and it may require placement of a provisional restoration which undermines the structural integrity of the tooth and propagates the tooth crack. Additional methods include bite tests using for example orange wood sticks and cotton wool rolls, or microscopic examination to detect tooth cracks. A disadvantage of the microscopic examination may be the cost and qualification needed to perform the method.

Current ways of identifying cracks also include use of X-ray imaging, near-infrared (NIR) transillumination, optical coherence tomography (OCT), indocyanine green near-infrared fluorescence, ultrasound, quantitative light-induced fluorescence (QLF), magnetic resonance imaging (MRI) and other.

The challenge remains to effectively identify and classify detected tooth cracks, to provide tooth crack details such as its depth and length, to provide a solution for monitoring tooth cracks over time.

Development of intraoral scanning techniques has been instrumental in the transition to modern digital dentistry. Use of an intraoral 3D scanner allows the dental practitioners to accurately and quickly capture intraoral situation of the patient which may then be visualized on a display as a digital 3D dental model. Obtained digital 3D dental model of patient's teeth may be used for diagnostic purposes to detect and visualize to the user, for example the dental practitioner, presence of various types of dental conditions.

The present disclosure addresses prospects to utilize capabilities of the intraoral 3D scanner systems to detect tooth cracks on the digital 3D model of the dental situation.

Beyond just presence of tooth cracks in the dental situation, there is also a need for accurate classification of tooth cracks and determining crack properties such as its length or depth.

### Summary

In an embodiment, a computer-implemented method for detecting tooth cracks on a digital 3D model of a dental situation is disclosed, the method comprising:
- receiving the digital 3D model of the dental situation,
- generating an input, from the digital 3D model, for a trained neural network for tooth crack detection, wherein the input comprises at least a part of the digital 3D model, the at least part of the digital 3D model comprising associated color information,
- detecting a tooth crack on the at least part of the digital 3D model by applying the trained neural network to the generated input,
- determining a severity level of the tooth crack, and
- displaying the at least part of the digital 3D model and the detected tooth crack.

In a further embodiment, a computer-implemented method for detecting tooth cracks on the digital 3D model of the dental situation is disclosed, the method comprising:
- receiving the digital 3D model of the dental situation,
- generating the input for the trained neural network for tooth crack detection, wherein the input comprises the at least part of the digital 3D model with associated color information,
- detecting the tooth crack on the at least part of the digital 3D model by applying the trained neural network to the generated input,
- determining the severity level of the tooth crack based on fluorescence information associated with the at least part of the digital 3D model, and
- displaying the at least part of the digital 3D model, the detected tooth crack and the severity level of the detected tooth crack.

Expression "3D" throughout the present disclosure refers to the term "three-dimensional". Similarly, term "2D" refers to the term "two-dimensional". Term "digital 3D model of a dental situation" refers to a digital, three-dimensional, computer-generated representation of the patient's dental situation.

Such digital 3D model may accurately correspond to the actual dental situation so that dental objects like teeth, gingiva, restorations and/or braces on the digital 3D model correspond to those of the dental situation.

The digital 3D model may be constructed by the processor of an intraoral scanner system, based on scan data collected in an intraoral scanning process. In the intraoral scanning process an intraoral 3D scanner may be used to scan the patient's dental situation comprising teeth and gingiva. The intraoral 3D scanner throughout the disclosure may also be referred to as the intraoral scanner. The digital 3D model can be stored in a memory of a computer system, for example in a Standard Triangle Language (STL) format or any other format suitable for displaying or printing 3D objects.

The method according to an embodiment may comprise receiving, by the processor, the digital 3D model of the dental situation.

The digital 3D model can be received or accessed by the processor. The digital 3D model may usually be displayed on a display screen in form of a 3D mesh, representing surfaces of teeth and gingival tissue of the dental situation. The 3D mesh may be comprised of individual facets, for example triangular facets while each facet comprises, for example three mutually connected vertices. Alternatively, the digital 3D model may be displayed as a point cloud comprising points, a graph comprising nodes and edges, a volumetric representation comprising voxels, or any other suitable 3D representation form.

Further, the method may comprise generating the input for the trained neural network for tooth crack detection, wherein the input comprises the at least part of the digital 3D model with associated color information. The at least part of the digital 3D model may be a tooth, a part of the tooth and/or a plurality of teeth. The digital 3D model may be realistic in that it comprises colors resembling the true dental situation. This color information may be captured by the intraoral scanner and used to generate the digital 3D model. The at least part of the digital 3D model, which may serve as the input for the trained neural network, may thus comprise associated color information which may aid in detecting tooth cracks.

The color information may comprise a Red-Green-Blue (RGB) color intensity values of the at least part of the digital 3D model. The color information present in the input may be beneficial as it facilitates identifying tooth cracks due to resulting variation in color of the enamel. The difference in color of the enamel may indicate a tooth crack presence. Thus, the color information may be of high relevance for the trained neural network in a process of detecting tooth cracks.

The color information may be in form of 2D color images that may be overlaid on the digital 3D model.

Depending on the type of the trained neural network, the input may be in 3D format or in 2D format. For example, one type of the trained neural network may be capable of processing 2D images of the digital 3D model while another type of the trained neural network may be capable of processing the digital 3D model in form of the point cloud. Thus, during the input generation, pre-processing operations may be performed such that the digital 3D model is adapted for evaluation by the trained neural network.

In an embodiment, the input may be in 3D format. An example of the input in 3D format may be a point cloud, a 3D mesh, a volumetric representation or a graph representation of the digital 3D model. It may be possible to transform the input from one 3D representation type, for example from the mesh representation, into another 3D representation type, for example the point cloud representation. A type of the trained neural network capable of processing the input in 3D format may be a trained PointNet network, or a PointNet-type network. These neural networks may be capable of processing the digital 3D model, or the at least part of the digital 3D model, in the point cloud form.

Generating the input may comprise sampling at least one input element of the at least part of the digital 3D model, or converting the at least one input element into a numerical form. The input element may be at least a point, a facet, a voxel, a node, a pixel, or more generally at least a unit element in representation of the digital 3D model. For example, one or more points from the point cloud representation of the digital 3D model may be converted into numerical form, for example a matrix of input values. The matrix may then form the input for the trained neural network.

If the digital 3D model is in the 3D mesh representation, it may be possible to convert the digital 3D model into the point cloud representation, by collapsing all vertices from the 3D mesh representation so that only points remain, thus forming the point cloud. The remaining point cloud may then be processed by the PointNet network, or the PointNet-type network.

In another embodiment, the input may be in 2D format such as a 2D image or a plurality of 2D images of the at least part of the digital 3D model.

Generating the input, from the digital 3D model, for the trained neural network for tooth crack detection may comprise generating one or more 2D images, such as color images, of the at least one tooth surface of the at least part of the digital 3D model. The one or more 2D images may then form the at least one input element. Alternatively, the at least one input element may be a pixel belonging to the one or more 2D images. The one or more 2D images may be generated by means of a virtual camera.

The input to the trained neural network may additionally or alternatively comprise geometric information associated to the at least part of the digital 3D model. The geometric information may also be referred to as topological information and may comprise a depth information relating to the camera position, an angle between a facet normal and a camera direction, the facet normal and/or a curvature information. The term "camera" in this context relates to the virtual camera used to view the digital 3D model. The camera may be characterized by a virtual camera property, for example field of view (FOV) and/or focal length.

The method may comprise applying the trained neural network to the generated input. Once generated, the input may be provided to the trained neural network in order to obtain an output from the trained neural network. All information forming the input may be concatenated into a final input tensor.

Further, the method may comprise detecting the tooth crack on the at least part of the digital 3D model based on applying the trained neural network. Detecting the tooth crack may comprise detecting a presence of the tooth crack and/or the severity value of the tooth crack.

The trained neural network may, in its output, comprise a probability value representing the likelihood that the tooth crack is detected in the input. Further information about the detected tooth crack may be comprised in the output of the trained neural network. For example, the trained neural network may identify the position of the tooth crack on the affected tooth, classify the detected tooth crack in one of predetermined types of tooth cracks, assign the severity level to the detected tooth crack and/or estimate the length and/or the depth of the tooth crack.

In the method of an embodiment, the at least part of the digital 3D model and the detected tooth crack may be displayed. In this way the information about the tooth crack may be conveyed to the user. For example, the tooth crack may be highlighted on the at least part of the digital 3D model to visually distinguish it from the rest of the displayed digital 3D model. This may be done by coloring facets of the at least part of the digital 3D model corresponding to the tooth crack.

Detecting the tooth crack may comprise detecting a size (length and/or width) of the tooth crack, a position of the tooth crack and/or a degree of confidence.

The size (length or width) of the tooth crack may be expressed in millimeters. The position of the tooth crack may be, for example a tooth identification number. The position of the tooth crack, in one example, may relate to marked facets representing the tooth crack on the at least part of the digital 3D model. The degree of confidence may indicate the confidence in the output prediction, for example based on training data that the trained neural network has been trained on, or based on test data that the trained neural network has been tested on. The degree of confidence may be displayed, for example as a number or as a color overlay where different colors are used to represent different degrees of confidence. The degree of confidence may, in an example, be a probability value provided by the trained neural network.

In an example, the degree of confidence may be determined based on analysing training data used for training of the trained neural network for tooth crack detection. In this way under-represented cases, referred to also as "special" cases may be identified in the training data. By identifying these cases, the "weak points" of the trained neural network for tooth crack detection may be identified. The degree of confidence may thus indicate that an input sample to the trained neural network for identifying tooth cracks may be of a special case type and that the trained neural network has not been sufficiently trained on that type of input.

Analysing the training data may comprise determining a distribution of the training data.

The distribution of the training data may be a distribution of the training samples according to a number of teeth per training sample. In this way digital 3D models that are under-represented in the training data may be identified. For example, the training data may have few training samples with 5 teeth or less. This may be a hint that, if the trained neural network for tooth crack detection is applied to a case (a 3D model) with 5 teeth, the output of the trained neural network for identifying the dental condition may have a low associated degree of confidence.

The distribution of the training data may be a distribution of the training samples according to, for example, presence of retainers, braces, preparations, fillings, restorations and/or third molars. By determining the distribution of the training data an outlier in the training data may be identified.

The outlier may be training sample comprising retainers, brackets, a preparation for a restoration, a filling, a restoration, a third molar, and/or a number of teeth less than a threshold.

In one example, a latent space representation of the outlier may be generated. Generating the latent space representation of the outlier may comprise encoding the outlier into a neural network such as an autoencoder network.

A latent space representation may be generated for every outlier that has been identified. In this way, a plurality of clusters in the latent space may be obtained, where each cluster of the plurality of clusters represents a type of under-represented training sample in the training data. The generated latent space representations may thereby represent a "list" of special cases that may affect the degree of confidence.

The latent space representation of the digital 3D model that is input into the trained neural network for tooth crack detection may be generated. Furthermore, the latent space representation of the digital 3D model may be compared to the latent space representation of the outlier to obtain a similarity measure. The similarity measure may be a linear distance of the latent space representation of the digital 3D model to the latent space representation of the outlier, or a linear distance of the latent space representation of the digital 3D model to the latent space representation of the cluster representing the outlier.

The degree of confidence may be assigned based on the obtained similarity measure.

The method according to an embodiment may further comprise:
- applying the trained neural network for tooth crack detection to test data comprising a plurality of test samples,
- identifying a test sample from the plurality of test samples satisfying a criterion,
- determining the degree of confidence based on the correspondence of the test sample and the digital 3D model that is input into the trained neural network for tooth crack detection.

The criterion may comprise determining that a percentage of the area of the test sample correctly classified is below a threshold. The test sample may be stored in a confidence score list.

A performance plot may be generated during application of the trained neural network for tooth crack detection to the test data and a latent space cluster for part of the test data resulting in poor performance may be generated. The performance may be judged, for example, by determining a percentage of correctly classified area test samples and comparing the percentage to a set tolerance. For example, good performance may be assigned if the percentage of a correctly classified area is over 98% for 90% of test samples in the test data.

Test data may be used to evaluate the performance of the trained neural network for tooth crack detection. The test data may comprise test samples. A test sample may be a digital 3D model of a dental situation or a part of a digital 3D model of a dental situation.

In an embodiment, a quality measure for test performance of the trained neural network for tooth crack detection may be determined. The test performance of the trained neural network may relate to performance of the trained neural network based on the test data. The quality measure for test performance may then be used to determine the degree of confidence. For example, if the quality measure for test performance is below a threshold, then the degree of confidence may be assigned a low value.

The latent space representation of the digital 3D model that is input into the trained neural for tooth crack detection may be generated. The latent space representation of the digital 3D model may be compared to the latent space cluster to obtain the similarity measure. The degree of confidence may be based on the obtained similarity measure.

Detecting the tooth crack may comprise detecting a tooth surface where the tooth crack is present, such as a labial surface, a buccal surface, a lingual surface, a distal surface, a mesial surface, an occlusal surface and/or a proximal surface between two teeth.

Detecting the tooth crack may comprise detecting a propagation direction of the tooth crack, such as a vertical direction (in direction of a tooth root towards the tooth crown), a horizontal direction or a slanting (oblique) direction.

Detecting the tooth crack may further comprise detecting an extension level of the tooth crack such as sub-gingival level, involving-incisal-edge level or gingival margin level.

Detecting the tooth crack may further comprise detecting a depth of the tooth crack. The depth of the tooth crack may be expressed in millimeters. Additionally or alternatively, the depth of the tooth crack may be expressed in text form, such as "limited to enamel", "extending to dentin" or "extending to pulp".

Detecting the tooth crack may comprise classifying the detected tooth crack into a type of tooth crack such as a craze line, a cracked tooth, a fractured cusp, a split tooth and/or a vertical root fracture.

The at least part of the digital 3D model may further comprise fluorescence information. This information may aid the process of detecting tooth cracks, determining severity levels of tooth cracks and/or classifying the tooth cracks into a tooth crack type.

The fluorescence information may comprise a red (R) fluorescence signal value and a green (G) fluorescence signal value for the at least part of the digital 3D model. The fluorescence information may be represented numerically in Red, Green, Blue (RGB) color space [R: 0-255; G: 0-255; B: 0]. No blue data component is available due to filtration of this component during scanning or during post-processing of the acquired scan data.

The fluorescence information may be in form of 2D fluorescence images that may be overlaid on the digital 3D model.

The fluorescence information may be recorded by means of the intraoral scanner which may be equipped with a light source capable of exciting fluorescent material in the dental situation, an image sensor capable of recording the emitted fluorescence, and a filter to block the excitation light from the sensor while transmitting the fluorescent light thereby allowing observance of fluorescence. Bacteria present on teeth of the dental situation is such fluorescent material emitting fluorescence when illuminated with the light source.

Therefore, the intraoral scanner, besides having an optical system to capture geometric information related to 3D space placing of objects in the dental situation and color information of the dental situation, may also be equipped with means to capture fluorescence emitted from the teeth in the dental situation. This may be achieved by integrating, in the intraoral scanner, the light source that can emit light intended to excite fluorescence in parts of the dental situation, for example a blue LED emitting light with wavelength of 405 nanometers, and the image sensor that can measure that emitted fluorescence. By scanning the dental situation with such an intraoral scanner, fluorescence information may be obtained.

The fluorescence information may, in an example, be comprised in each vertex and/or facet of the at least part of the digital 3D model represented as the 3D mesh.

The fluorescence information may be assigned to the digital 3D model based on a plurality of images taken by the intraoral scanner from a variety of angles. A weighting system may be applied when assigning the fluorescence information to the digital 3D model such that lower weights are assigned when a surface is seen from a steep angle.

The fluorescence information may be used as part of the input to the trained neural network. This additional information in the input may result in better performance of the trained neural network.

Alternatively or additionally, the output of the trained neural network, namely the detected tooth crack, may be classified into a severity class based on the fluorescence information. Thus determining the severity level of the tooth crack may be based on the fluorescence information.

The method may comprise computing a bacteria presence score indicating an amount of bacteria present in the detected tooth crack. The bacteria presence score may be determined based on the fluorescence information. The bacteria presence score provides deeper insight into what the severity of the detected tooth crack is, and thereby provides a basis for accurate treatment planning.

The bacteria presence score may be determined based on a function of the red (R) fluorescence value and/or the green (G) fluorescence value of the fluorescence information. For example, difference R-G may be calculated for every facet of the detected crack and optionally for the facets neighbouring the facets of the detected crack. Calculated difference values R-G may be averaged to determine the bacteria presence score. Another example of a function of the red (R) fluorescence value and/or the green (G) fluorescence value of the fluorescence information is a ratio (R-G)/(R+G).

The at least part of the digital 3D model may further comprise infrared image data. This image data may comprise a plurality of 2D images of the dental situation, for example acquired with the intraoral scanner equipped with near-infrared (NIR) light source emitting light in a near-infrared spectrum of 700-2500 nm, for example a light emitting diode (LED) emitting light at 940 nm. Infrared image data may also be referred to as near-infrared image data, infrared images or near-infrared images.

Tooth cracks may be seen in the infrared images as lines separating bright and dark parts of a tooth. Specifically, in case of severe tooth cracks the light transmission through the scattering denting material is affected such that parts of varying brightness may be observed. Transmission of light emitted from the NIR light source through the tooth material affected by the tooth crack may result in appearance of a shadow downstream of the tooth crack along the angle of illuminating NIR light. This shadow may be seen as a dark part of the tooth and corresponds to the tooth crack.

The infrared image data of the dental situation may comprise the shadow corresponding to the tooth crack. This shadow may be detected in the infrared image data and may be used as part of the input to the trained neural network to improve tooth crack detection.

The infrared image data may be used as part of the input to the trained neural network. Thus in an embodiment the input to the trained neural network may further comprise the infrared image data. Alternatively or additionally the infrared image data may be used to post-process the output of the trained neural network to gain further insight into the detected tooth crack, such as to determine the depth of the detected tooth crack and/or the length of the detected tooth crack.

The method may comprise determining the length and/or the depth of the detected tooth crack based on the infrared image data.

For example, the tooth crack may be detected as a result of applying the trained neural network to the generated input. Subsequently, a part of the infrared image data, for example a single infrared image, corresponding to the detected tooth crack may be identified. Furthermore, the shadow in the part of the infrared image data corresponding to the detected tooth crack may be identified, and the length and/or the depth of the detected tooth crack may be determined based on the identified shadow.

In an embodiment, the input to the trained neural network may further comprise a modified near-infrared image, wherein the modified near-infrared image is obtained by mutually subtracting a first near-infrared image and a second near-infrared image, wherein the first near-infrared image depicts a tooth illuminated by a near-infrared light source only through a first surface, wherein the second near-infrared image depicts the tooth illuminated by a near-infrared light source only through a second surface, and wherein the first surface and the second surface are different approximal surfaces of the tooth.

In an embodiment, the at least part of the digital 3D model may comprise combined image data, wherein the combined image data may be a function of the infrared image data, the fluorescence information and/or the color information.

Combined image data may be generated to enhance contrast of near-infrared images and thereby to improve detection of the tooth cracks. The visibility of tooth cracks is enhanced by generating the combined images, compared to observing the same tooth cracks in the infrared image data, color images and/or fluorescence images. Combined images may offer better contrast between the dental cracks and the rest of tooth structure such as enamel or dentin.

Combined images may be generated by combining intensity levels of pixels of the image sensor in the intraoral scanner, the pixels being associated with different wavelengths. For example, at a first time period, the image sensor unit may capture light information that relates to visible wavelength, for generating color information. At a second time period, the image sensor unit may capture light information that relates to a near-infrared wavelength, for generating infrared image data. The intensity levels of the two time periods may be recorded and combined for enhancing the visibility of dental conditions. The combination of the intensity levels may be done digitally by subtraction and/or addition of the intensity levels. In another example, the intensity levels may be captured and recorded during at least three time periods for at least three different wavelengths, such as white-coloured wavelength, blue-coloured wavelength and near-infrared wavelength.

The input to the trained neural network may comprise, exclusively or in a combination, the combined image data. An advantage to utilizing combined image data as the only input to the trained neural network is that the performance of the trained neural network is improved, as processing may be limited to one type of data, namely the combined image data, compared to processing the infrared image data, the fluorescence information and the color information as separate inputs.

In an embodiment, the input to the trained neural network may comprise the combined image data. Alternatively or additionally, the method may comprise utilizing the combined image data to post-process the output of the trained neural network. For example, the method may comprise determining the length and/or the depth of the detected tooth crack based on the combined image data.

The method may further comprise receiving a digital measurement of a patient's bite showing distances between teeth of the patient's jaws and contact points of the teeth of the patient's jaws. This type of digital measurement may be obtained with the intraoral scanner.

The method may further comprise determining if a tooth comprising the detected tooth crack is in contact with an antagonist tooth based on the received digital measurement of the patient's bite. An alarm may be displayed based on determining that the tooth comprising the detected tooth crack is in contact with the corresponding antagonist tooth. In this case, the tooth crack may be in danger of being pathogenic, experiencing higher load more often. The tooth comprising detected tooth crack in contact with the antagonist tooth may be displayed on the digital 3D model.

The method may further comprise receiving a digital measurement of the patient's biting force. This measurement may be obtained by recording the patient biting on existing measurement devices capable of recording occlusal contacts, biting force and/or relationship of occlusal surfaces.

In an embodiment, the input to the trained neural network may comprise the digital measurement of the patient's biting force. In this case the output of the trained neural network may detect and/or classify the tooth crack with a higher accuracy.

The digital measurement of the patient's biting force may alternatively be used to post-process the output of the trained neural network. In an example, the method may comprise determining if the detected tooth crack is in contact with an antagonist tooth based on the received digital measurement of the patient's biting force. In this case, the tooth crack may be in danger of being pathogenic, experiencing higher load more often. The method may further comprise displaying an alarm based on determining that the detected tooth crack is in contact with an antagonist tooth.

The method may further comprise displaying the detected tooth crack in contact with the antagonist tooth on the digital 3D model.

The method may further comprise recording obtained information about the detected tooth crack in a dental record of the patient such as a dental chart. Thus, the location of the tooth crack may be recorded, for example in form of affected tooth number and/or tooth surface. Moreover, size (length and/or depth) of the tooth crack, severity level of the tooth crack and/or type of the tooth crack may be recorded.

The method according to an embodiment may further comprise:
- receiving a further digital 3D model representative of the dental situation at a later visit to a dental clinic,
- generating the input for the trained neural network for tooth crack detection comprising at least a part of the further digital 3D model with associated color information,
- detecting a corresponding tooth crack on the at least part of the further digital 3D model by applying the trained neural network to the generated input.

The further digital 3D model may be understood as a follow-up scan of the same patient at a later time. The corresponding tooth crack on the further digital 3D model is thus the same tooth crack detected on the at least part of the digital 3D model previously analyzed.

Furthermore, the method may comprise displaying the tooth crack and the corresponding tooth crack simultaneously, for example on the display device. In an example, this method may comprise displaying a tooth suffering from the tooth crack and a corresponding tooth suffering from the corresponding tooth crack in a mutually superimposed state. The corresponding tooth and the tooth may have the same Universal Numbering Notation (UNN) number.

Parameters such as length and/or depth of the corresponding tooth crack may be determined in same or similar fashion as for the tooth crack of earlier scan. The method may comprise determining a difference between the length of the tooth crack and the length of the corresponding tooth crack. Alternatively or additionally, the method may comprise determining a difference between the depth of the tooth crack and the depth of the corresponding tooth crack.

In an embodiment, detecting the tooth crack may comprise determining, by the trained neural network, a curve representing the tooth crack. The curve may be a spline. The spline may be determined on the at least part of the digital 3D model. Determining the spline may be an efficient way to represent the tooth crack, as the spline can accurately conform to the shape of the tooth crack. Furthermore, the length of the spline may be computed to determine the length of the tooth crack.

In a different embodiment, detecting the tooth crack may comprise determining a number of facets of the at least part of the digital 3D model representing the tooth crack. The method may further comprise computing length of a line within the determined number of facets in order to determine the length of the tooth crack. The line may be a line connecting midpoints of the determined facets and may thus represent the length of the tooth crack. When the tooth crack is detected, the corresponding facets may be marked completely. This method may not be ideal for representing the tooth crack as the facets often do not form a line and/or can vary in their size. In this respect, representing the tooth crack as the spline may be advantageous.

The trained neural network may be trained on training data comprising a plurality of training samples. These training samples may be separate digital 3D models of dental situations where tooth cracks have been labelled, for example manually or automatically. The training samples may thus be annotated for tooth cracks presence, location, type and/or severity.

The training data may comprise a training sample of a dental situation with a disclosed tooth crack.

The training sample with the disclosed tooth crack may be obtained by scanning a corresponding dental situation by means of an intraoral scanner, wherein a disclosing agent is infiltrated in the tooth crack and wherein the disclosing agent is subsequently removed from tooth surfaces of the corresponding dental situation. Thereby, the disclosing agent may only be present in the dental crack which may then be scanned.

A training process for a neural network for tooth crack detection may comprise:
- obtaining the training data, the training data comprising the training sample with the disclosed tooth crack and a corresponding second training sample without the tooth crack disclosed,
- generating target data by identifying the tooth crack disclosed on the first training sample and transferring the identified tooth crack onto the second training sample,
- inputting the second training sample into the neural network to obtain the output from the neural network,
- comparing the output from the neural network to the generated target data to obtain the loss,
- adjusting weights of the neural network based on the obtained loss, and
- repeating the inputting step until the loss satisfies the stopping criterion.

An advantage of this type of training method is that the training data does not need to be annotated manually. Instead, information on tooth crack presence is automatically extracted from scans where tooth cracks are disclosed by means of the disclosing agent, and transferred to corresponding scans without disclosed tooth cracks.

The training sample and the corresponding training sample may be the same digital 3D models, where the tooth crack is disclosed in the training sample and wherein the same tooth crack is not disclosed in the corresponding second training sample.

Identifying the tooth crack disclosed on the first training sample may comprise:
- comparing a color of a facet of the first training sample to a reference color representing the disclosing agent to compute an Euclidian distance between the color of the facet and the reference color.

The facet may be assigned to a disclosing agent mask if the Euclidian distance is lower than a color distance threshold.

In another example, the trained neural network may be trained on training data comprising a training sample of a dental situation, wherein a tooth crack in the training sample is disclosed using reference data. The reference data may comprise high-resolution photographs of the dental crack in the first annotated training sample.

In an embodiment, a computer-implemented method for providing treatment recommendation for a detected tooth crack is disclosed, the method comprising:
- receiving a digital 3D model of a dental situation, wherein the digital 3D model comprises associated fluorescence information,
- receiving an indication of the detected tooth crack corresponding to a part of the digital 3D model,
- evaluating fluorescence information corresponding to the part of the digital 3D model,
- generating a recommendation for treatment of the detected tooth crack based on evaluated fluorescence information.

Evaluating fluorescence information may comprise determining a bacteria presence score corresponding to the part of the digital 3D model based on a red fluorescence value of the fluorescence information.

The indication of the detected tooth crack may be obtained using X-ray imaging, near-infrared transillumination, optical coherence tomography, ultrasound, quantitative light-induced fluorescence, periodontal probing, bite tests, microscopic examination and/or magnetic resonance imaging.

The recommendation for treatment of the detected tooth crack may comprise a recommendation for a root canal treatment, a recommendation for a vital pulp therapy, a recommendation for creating a filling, a recommendation for introducing a crown, a recommendation for removing a part of a corresponding tooth, a recommendation for removing the corresponding tooth and/or a recommendation for an endodontic surgery.

The method may further comprise displaying a video tutorial with examples on how to interpret the fluorescence information.

Further, the method may comprise displaying a user manual comprising steps for identifying tooth cracks based on the fluorescence information.

A virtual assistant may further be displayed, the virtual assistant configured for guiding the user to identify tooth cracks based on the fluorescence information.

The virtual assistant may be a chat bot, a pop-up information text, a voice guide and/or an indication of a right or wrong interpretation.

The virtual assistant may be displayed upon detecting a user interaction such as movement of a mouse/touch pad, a click of a button and/or a voice control.

Furthermore, a digital measurement tool for measuring the tooth crack may be displayed.

In an embodiment a dental system for detecting tooth cracks is disclosed, the system comprising:
- an intraoral scanner with a near-infrared light source, the intraoral scanner configured to:
- illuminate a dental situation with light from the near-infrared light source,
- acquire an image of the dental situation based on the illuminated light, wherein a disclosing agent is first applied to the teeth of the dental situation and subsequently removed from surfaces of the teeth, further wherein the disclosing agent is configured to absorb the light from the near-infrared light source,
   the system further comprising a processor configured to:
- detect a change in brightness of a tooth surface in the image, wherein the change in brightness is due to the disclosing agent absorbing the light from the near-infrared light source, and
- detect the tooth crack in the image based on the detected change in brightness.

The processor may further be configured to measure a length of the shadow of the tooth crack and determine that the tooth crack is a craze line based on the measured length.

The processor may further be configured to measure the length of the shadow of the tooth crack and determine that the tooth crack is an enamel-dentin crack based on the measured length.

The processor may further be configured to measure the depth of the tooth crack based on the length of the shadow of the tooth crack.

In an embodiment, a computer-implemented method for detecting tooth cracks is disclosed, the method comprising:
- receiving scan data of a patient's tooth suffering from the tooth crack, wherein the scan data is representative of the patient's tooth while the patient is biting on a tip of the intraoral scanner with the tooth suffering from the tooth crack,
- recording changes associated with the tooth crack in the patient's tooth,
- displaying the recorded changes.

The recorded changes may relate to a severity level of the tooth crack.

Recording changes associated with the tooth crack in the patient's tooth may comprise recording changes between two-dimensional images in the scan data.

The changes associated with the tooth crack in the patient's tooth may relate to the tooth crack opening up.

In a further example, recording changes associated with the tooth crack in the patient's tooth may comprise recording pressure exerted by the tooth suffering from the tooth crack while the patient is biting on the tip of the intraoral scanner.

The tip of the intraoral scanner may comprise an outer metallic frame. In this way sufficient structural base may be provided for the patient to bite on the intraoral scanner. The tip of the intraoral scanner may comprise a pressure sensor in the outer metallic frame.

A computer program product is disclosed comprising instructions which, when the program is executed by a computer, causes the computer to carry out any one or more methods according to the disclosure.

A computer readable medium is further disclosed comprising instructions which, when executed by a computer, cause the computer to carry out any one or more methods according to the disclosure.

A data processing apparatus is further disclosed comprising means for carrying out any one or more methods according to the disclosure.

A dental scanning system is further disclosed comprising means for carrying out any one or more methods according to the disclosure.

### Brief description of the figures

Aspects of the disclosure may be best understood from the following detailed description taken in conjunction with the accompanying figures. The figures are schematic and simplified for clarity, and they just show details to improve the understanding of the claims, while other details are left out. The individual features of each aspect may each be combined with any or all features of other aspects. These and other aspects, features and/or technical effects will be apparent from and elucidated with a reference to the illustrations described hereinafter in which:
FIGS. 1A-1E illustrate various types of tooth cracks;
FIG. 2 is a flow chart illustrating a method for detecting tooth cracks according to an embodiment;
FIG. 3 illustrates a type of the trained neural network for detecting tooth cracks with an example of the input and the output;
FIG. 4 illustrates a further type of the trained neural network for detecting tooth cracks;
FIG. 5 illustrates comparison of a detected tooth crack on a baseline scan and the same tooth crack on a follow-up scan;
FIGS. 6A-6C illustrate distinguishing various severity levels of the detected tooth crack based on fluorescence information;
FIGS. 7A-7C illustrate detection of the tooth crack using infrared image data;
FIGS. 8A-8F illustrate detection of the tooth crack while biting on an intraoral scanner and examples of intraoral scanners suitable for detecting the tooth crack while biting;
FIG. 9 illustrates detecting tooth crack characteristics based on a digital measurement of the patient's biting force;
FIG. 10 is a flowchart illustrating a method for training a neural network for detecting tooth cracks;
FIG. 11 illustrates a dental scanning system according to the disclosure;
FIG. 12 illustrates an example of a computer architecture configured for carrying out a method according to the disclosure.

### Detailed description

In the following description, reference is made to the accompanying figures, which show by way of illustration how the invention may be practiced.

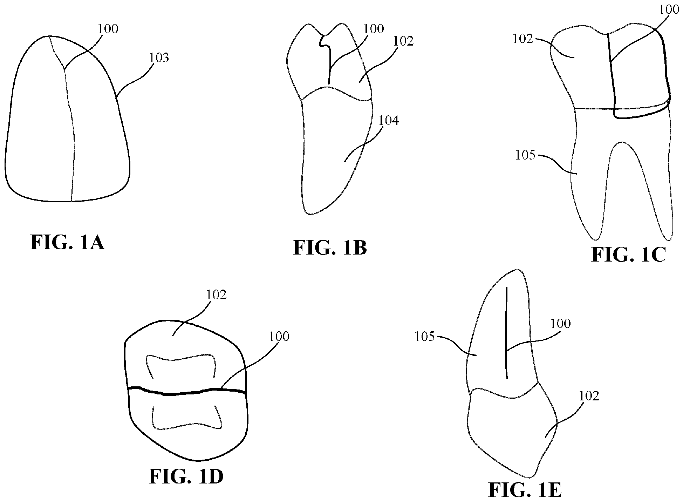

FIGS. 1A-1E illustrate various types of tooth cracks 100. FIG. 1A illustrates a craze line on a labial surface of an incisor tooth 103. Craze lines are micro-fractures of the enamel only, present in teeth of most of adults. Craze lines commonly appear on anterior teeth. In posterior teeth, they usually extend along the buccal and lingual surfaces thereby crossing either cusp ridges or grooves. In the presence of a dental filling, craze lines may often be observed around the dental filling.

Since the craze line only affects the enamel, it causes no pain to the patient. Craze lines are the only type of tooth crack 100 not needing any treatment.

Unfortunately, the craze line can easily be confused with another type of the tooth crack 100 called a cracked tooth, shown in FIG. 1B, which requires treatment. It is therefore crucial to be able to distinguish between these two types of tooth cracks.

The cracked tooth, shown in FIG. 1B, may be defined as an incomplete fracture initiated from a crown 102 of a tooth and may extend sub-gingivally. This type of tooth crack 100 is usually in a mesial-distal direction. The cracked tooth may extend through one marginal ridge or may extend through both proximal surfaces. The vertical depth of the crack is also variable. The cracked tooth is in general more centred and apical than a fractured cusp for example (shown in FIG. 1C), resulting in increase in the probability of causing pulpal and periapical pathosis as it extends apically. The cracked tooth in FIG. 1B is shown not extending into the root 104.

FIG. 1C illustrates the fractured cusp. The fractured cusp is defined as a complete or incomplete fracture of the crown 102 of the tooth extending sub-gingivally. It is usually directed both mesiodistally and buccolingually. The fracture usually involves two aspect of the cusp by crossing the marginal ridge and also extending down a buccal or lingual groove. The fractured cusp may be treated depending upon the amount of remaining tooth structure. Further treatments may be needed in the event that the crack affects the pulp chamber of the tooth.

FIG. 1D illustrates a split tooth. The split tooth may be defined as a complete fracture initiated from the crown 102 extending sub-gingivally. It typically extends through both marginal ridges and the proximal surfaces to the proximal root. The split tooth is the end result of the cracked tooth (cracked tooth shown in FIG. 1B). In the case of the split tooth there are no dentin connections, tooth segments are entirely separated. The split tooth may occur suddenly, but is typically the result of the long-term growth from an incomplete tooth crack.

FIG. 1E illustrates a vertical root fracture. The vertical root fracture may be a complete or incomplete fracture of the root 105 extending from coronal to apical, splitting the root 105 into two parts, namely buccal and lingual. The vertical root fracture may involve one or both proximal surfaces, only buccal, only lingual, or both buccolingual surfaces. The vertical root fracture may extend along the length of the root 105 or as a shorter segment along any portion of the root 105. There may or may not be patient symptoms associated with the vertical root fracture and it thus may go unnoticed until periapical pathosis occurs. If discovered they are usually discovered on routine periapical x-rays.

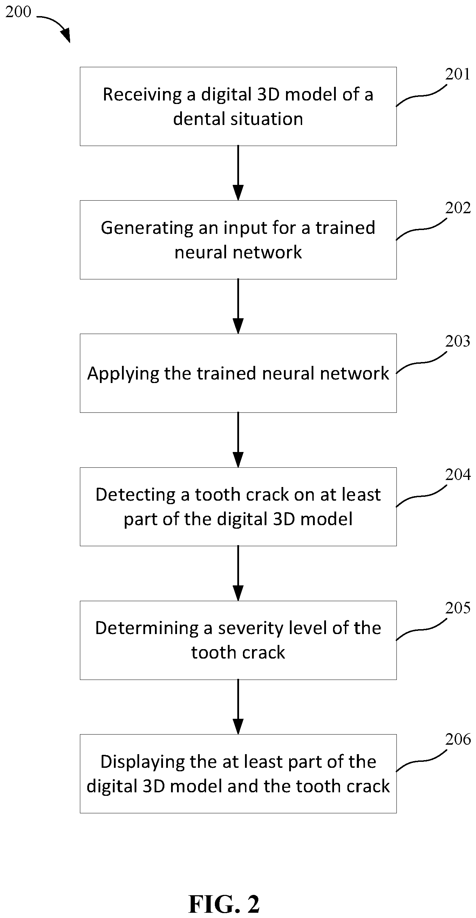

FIG. 2 is a flow chart illustrating a method 200 for detecting tooth cracks on a digital 3D model of a patient's dental situation, according to an embodiment.

The digital 3D model may be received, by a processor, in step 201. The digital 3D model may be stored in a memory of a computer system, for example in a Standard Triangle Language (STL) format or in any other suitable format for storing or printing 3D objects. The digital 3D model may be received by the processor, for example when a user engages a button in a user interface of a dental scanning system 1100. The digital 3D model may usually be displayed on a display screen in the form of a 3D mesh, a point cloud, a 3D graph, a volumetric representation, or any other suitable 3D representation form.

In step 202, generating an input 301, 401 for a trained neural network 300, 400 may be performed. The input 301, 401 may be generated from the received digital 3D model. For example, at least a part of the digital 3D model, such as a single tooth or a single tooth surface, may be input to the trained neural network 300, 400 in order to determine whether the tooth crack 100 is present and/or to classify the tooth crack 100. The at least part of the digital 3D model may thus be a tooth, a plurality of teeth, a tooth surface, a part of a tooth and/or a jaw. In other cases, the complete digital 3D model may be processed by the trained neural network 300, 400.

The at least part of the digital 3D model, serving as the input 301, 401 for the trained neural network 300, 400, may comprise associated color information. This information may be beneficial as it aids the trained neural network 300, 400 in detecting tooth cracks 100 in the input 301, 401.

Color information, also referred to as natural color information, may relate to surface color of scanned teeth and/or gingiva of the dental situation and may be obtained through scanning of the dental situation of the patient by means of an intraoral scanner 801. This information may be expressed through Red-Green-Blue (RGB) color intensity values stored in the digital 3D model, for example in points, vertices or nodes of the digital 3D model. The presence of natural color information in the input 301, 401 may be beneficial for tooth crack detection as it may facilitate identifying tooth cracks 100 due to color variations in the cracked tooth area.

Step 202 of generating the input 301, 401 may comprise pre-processing of the digital 3D model in order to adapt it for the trained neural network 300, 400. For example, 2D images may be generated from the at least part of the digital 3D model if the trained neural network 300 is suitable for processing 2D image information. In another example, a point cloud representation of the digital 3D model in the 3D mesh format may be generated, for example by converting facets of the 3D mesh into the point cloud using facet midpoints. This conversion may be performed if the trained neural network 400 is suitable for processing point cloud information.

The trained neural network 300, 400 may be applied to the generated input 301, 401, as illustrated in the step 203. In other words, the input 301, 401 may be fed to the trained neural network 300, 400. Once the input 301, 401 is processed by the trained neural network 300, 400, the trained neural network 300, 400 may produce an output 305, 405 which may comprise an indication of the tooth crack 100 presence and/or type in the input 301, 401.

Step 204 illustrates detecting the tooth crack 101 on the at least part of the digital 3D model based on applying the trained neural network 300, 400. Detecting the tooth crack 100 may comprise detecting presence of the tooth crack 100 such as outputting a binary indication of whether the tooth crack 100 is detected, for example "Yes" or "No". Additionally or alternatively, detecting the tooth crack 100 may comprise classifying the tooth crack 100 in a tooth crack type, wherein the tooth crack type is one of the craze line, the cracked tooth, the fractured cusp, the split tooth or the vertical root fracture. Additionally or alternatively, detecting of the tooth crack 100 may comprise detecting a tooth crack parameter such as a length of the tooth crack 100, a depth of the tooth crack 100 and/or a severity level of the tooth crack 100. Additionally or alternatively, detecting the tooth crack 100 may comprise identifying facets on the at least part of the digital 3D model corresponding to the tooth crack 100.

In an embodiment detecting the tooth crack 100 may comprise detecting presence of the tooth crack 100 such as outputting the binary indication of whether the tooth crack 100 is detected, for example "Yes" or "No". The output 305, 405 of the trained neural network 300, 400 may then be post-processed, based on fluorescence data, the infrared image data and/or combined image data, for example to detect the severity level of the tooth crack 100.

Step 205 illustrates determining a severity level of the detected tooth crack 100 based on fluorescence information associated with the at least part of the digital 3D model. This may be done, for example, by computing a bacteria presence score indicating an amount of bacteria present in the detected tooth crack 100.

Step 206 illustrates displaying of the at least part of the digital 3D model, the detected tooth crack 100 and/or the severity level of the detected tooth crack 100. Displaying may occur on the display device, for example on a computer screen, a tablet or a mobile phone. For example, the detected tooth crack 100 may be displayed as a curve on the at least part of the digital 3D model. The curve may be any type of curve such as a line or a spline. The severity level may be visualized graphically by using different color overlays on the digital 3D model. For example, a severe tooth crack may be highlighted by coloring corresponding facets of the digital 3D model in a certain color (e.g. red or orange).

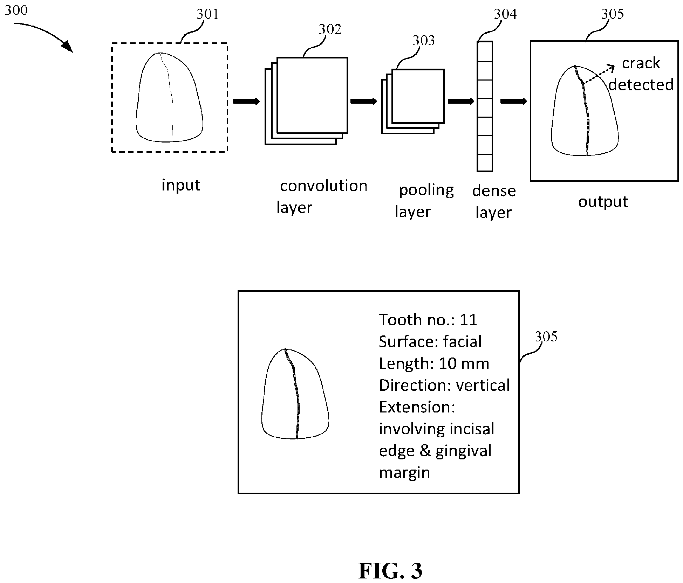

FIG. 3 illustrates a type of the trained neural network 300 for tooth crack detection.

The trained neural network 300 may be a convolutional neural network (CNN) and may comprise a plurality of convolution layers 302 capable of capturing low-level features of the input 301, i.e. obtaining convolved features. Moreover, one or more of pooling layers 303 may be present, responsible for reducing the spatial size of convolved features. Convolutional neural networks may be particularly suitable for processing matrix information such as 2D images, illustrated as the input 301.

The input 301 may be in a 2D format and may comprise a 2D image, or a part of the 2D image of the at least part of the digital 3D model. The input 301 may be a tooth or a surface of the tooth affected by the tooth crack 100.

The output 305 may comprise the at least one probability value representing the likelihood that the tooth crack 100 is present in the input 301 and/or may classify the tooth crack 100 further into the tooth crack type, for example one of the craze line, the cracked tooth, the fractured cusp, the split tooth, the vertical root fracture. The trained neural network 300 in this case performs a classification task.

In another example, the trained neural network 300 may perform segmentation task such that each element, for example each pixel, of the input 301, may be classified as representing the tooth crack 100 or healthy tooth structure.

In an example, shown in the bottom of the FIG. 3, the output 305 may comprise additional information such as a number of the affected tooth, a tooth surface where the tooth crack 100 is located, the length of the tooth crack 100, a propagation direction of the tooth crack 100 and/or an extension level of the tooth crack 100.

Another example of the trained neural network, particularly suitable for performing classification tasks, may be a trained Residual Network (ResNet) or a variation of it.

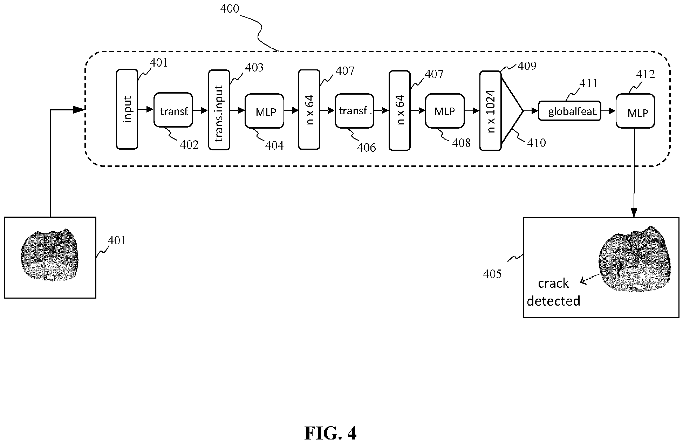

FIG. 4 illustrates a further example of the trained neural network 400 for detecting tooth cracks 100.

The trained neural network 400 may be a PointNet or a PointNet-like neural network capable of processing the at least part of the digital 3D model in form of a point cloud. Model presented in FIG. 4 is capable of taking an unordered point set as input. A generic model of this network architecture was developed by Stanford University ("PointNet: Deep Learning on Point Sets for 3D Classification and Segmentation", Charles R. Qi, Hao Su, Kaichun Mo, Leonidas J. Guibas). Additional to PointNet neural network, the trained neural network 400 may be a PointNet++ or a PointNet-like neural network (based on PointNet architecture).

A point cloud representation of a tooth of the digital 3D model may serve as the input 401 to the trained neural network 400. The input 401 may be of a format n x 3, where n is a number of 3D points representing the tooth and dimension 3 stands for three coordinates of each point representing the tooth. The input 401 may be processed by an input transformation block 402 which may be a 3 x 3 rotation transformation. The input transformation block 402 has the effect of achieving invariance under transformation. Block 403 may be a transformed input with the format n x 3. Alternatively, the input 401 may be of a format n x 6, where n is the number of 3D points representing the tooth and dimension 6 stands for three coordinates of each point representing the tooth and three coordinates representing a normal of each point.

Block 404 may represent a first multi-layer perceptron with shared weights, which may process independently all 3D points for feature extraction. Its function is to achieve invariance under permutation of 3D points order. Architecture of the first multi-layer perceptron 404 (MLP) may comprise an input layer of 3 dimensions, a hidden layer of 64 neurons while an output layer may comprise 64 features. Block 407 may have dimension of n x 64.

Feature transformation block 406 may comprise a transformation matrix of dimensions 64 x 64, which may be a regularization matrix, for example of L2 type. Block 407 may be dimensioned as n x 64. Block 408 represents a second multi-layer perceptron with shared weights. Architecture of the second multi-layer perceptron 408 (MLP) may comprise an input layer of 64 features, two hidden layers of 64 neurons and 128 neurons, while an output layer of the second multi-layer perceptron 408 may comprise 1024 features. Block 409 may have dimension of n x 1024. In max pooling block 410, feature vectors of 3D points are aggregated to create a 1024-dimensional global feature representation 411 of the input 401. Block 412 represents a third multi-layer perceptron. Architecture of the third multi-layer perceptron 412 (MLP) may comprise an input layer of 1024 features, two hidden layers of 512 neurons and 256 neurons, while an output layer of the third multi-layer perceptron 412, also referred to as the output 405 of the trained neural network 400, may comprise classification scores.

The output 405 of the trained neural network 400 may comprise an indication that the tooth crack 100 is present in the input 401 and/or may determine a location of the tooth crack 100 in the input 401. The output 405 may further classify the tooth crack 100 into a tooth crack type, for example one of the craze line, the cracked tooth, the fractured cusp, the split tooth, the vertical root fracture.

In another example the output 405 may comprise additional information such as the number of the affected tooth, the tooth surface where the tooth crack 100 is located, the length of the tooth crack 100, the propagation direction of the tooth crack 100, the severity value of the tooth crack 100 and/or the extension level of the tooth crack 100.

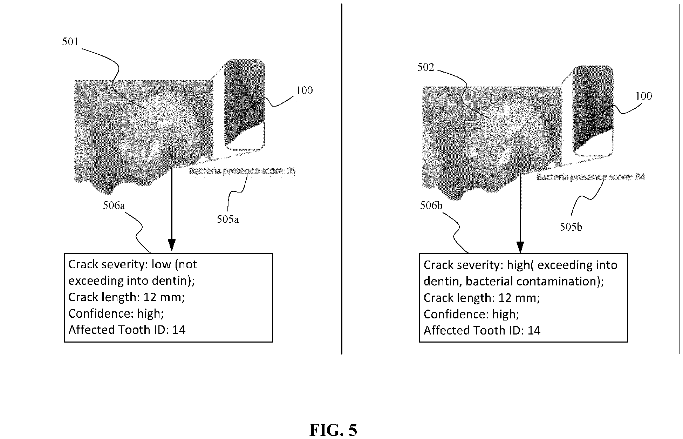

FIG. 5 illustrates comparison of the detected tooth crack 100 on the digital 3D model 501 and the same tooth crack 100 detected on the further digital 3D model 502 representative of the dental situation at a later visit to a dental clinic. The further digital 3D model 502 may be referred to as a follow-up digital 3D model 502.

The digital 3D model 501 and the further digital 3D model 502, or their parts, may be shown in a side-by-side view suitable for comparison as in FIG. 5. This side-by-side view may be displayed on the display of the computer 1110, for example. The digital 3D model 501 and the further digital 3D model 502 may also be shown in a superimposed state (not shown in figures), with a slider arranged to gradually hide one of the digital 3D model 501 or the further digital 3D model 502.

The tooth crack 100 on the digital 3D model 501 and a corresponding tooth crack 100 on the further digital 3D model 502 may be compared by visually highlighting the difference between the detected tooth cracks. For example, the facets representing the difference between the two detected tooth cracks may be colored in a different color (such as red) compared to the color of facets representing the non-changed part of the detected tooth cracks (such as black).

The tooth crack 100 on the digital 3D model 501 and a corresponding tooth crack 100 on the further digital 3D model 502 may be identified, for example by applying the trained neural network 300, 400 for detecting tooth cracks. This may comprise identifying parts of the digital 3D model 501 and the further digital 3D model 502, for example their facets, representing the tooth crack 100.

The output 305, 405 of the trained neural network 300, 400 may further be post-processed to determine a bacteria presence score 505a, 505b associated with the detected tooth crack 100. The bacteria presence score 505a, 505b may indicate an amount of bacteria present in the area of the detected tooth crack 100, for example in the facets identified to represent the tooth crack 100. This amount of bacteria presence may be correlated with the severity value of the detected tooth crack 100. Generally, the higher the amount of bacteria present, the higher the severity value is and the higher the potential for further tooth deterioration.

The at least part of the digital 3D model 501, 502 may comprise fluorescence information associated with fluorescence emitted from fluorescent material present on teeth of the dental situation.

Fluorescence information may comprise a red (R) fluorescence signal value and a green (G) fluorescence signal value for each unit element, such as a facet, a point, a vertex, of the at least part of the digital 3D model. The fluorescence information may therefore be represented in numerical form in Red, Green, Blue (RGB) color space.

The fluorescence information may be recorded by means of the intraoral scanner 801 equipped with a blue light source capable of exciting fluorescent material in the dental situation, an image sensor capable of recording the emitted fluorescence, and a filter to block the excitation light from the sensor while transmitting the fluorescent light. The bacteria present in and/or around the tooth crack 100 is such fluorescent material which emits fluorescence when illuminated with the blue light source with wavelength of around 400 nanometers, for example, 405 nanometers. The emitted fluorescence can then be observed.

The red fluorescence, indicating bacteria present in and/or around the tooth crack 100, may be detected and relevant information may be deduced from the detected fluorescence. A blue (B) fluorescence signal value may be filtered out during the scanning process or afterwards. The blue (B) fluorescence signal value may therefore be zero.

The detected tooth crack 100 may be classified based on the fluorescence information into different severity categories. For example, the bacteria presence score 505a, determined for the tooth crack 100 may have a value of 35. Based on this bacteria presence score 505a, the severity value of the tooth crack 100 may be determined and displayed, for example in an information window 506a.

The bacteria presence score 505a, 505b may be determined based on the red (R) fluorescence signal value and/or the green (G) fluorescence signal value of the fluorescence information. Various functions of R and G values may be used, for example a difference signal R-G or a ratio (R-G)/(R+G).

A function of R and/or G values may be computed for all facets representing the tooth crack 100. An average or a weighted average of all computed values may then be determined to obtain the bacteria presence score 505a, 505b.

The bacteria presence score 505b may be determined for the tooth crack 100 detected in the at least part of the further digital 3D model 502 in a similar manner, based on the fluorescence information of the at least part of the further digital 3D model 502.

As shown in FIG. 5, the detected tooth crack 100 may have the associated bacteria presence score 505b value of 84 in the follow-up scan. This value may be sufficiently high to determine that the severity level of the tooth crack 100 is high, as indicated in the information window 506b.

Threshold values for the bacteria presence score may be set up to classify the tooth cracks, for example in three categories: low, medium and high. The threshold values may be empirically determined. The threshold values for the bacteria presence score may be adjustable by the user or may be pre-set in the computer program product of the disclosure.

Instead, or in addition to, using the fluorescence information to post-process the output 305, 405 of the trained neural network 300, 400, the fluorescence information may be used as part of the input 301, 401 to the trained neural network 300, 400.

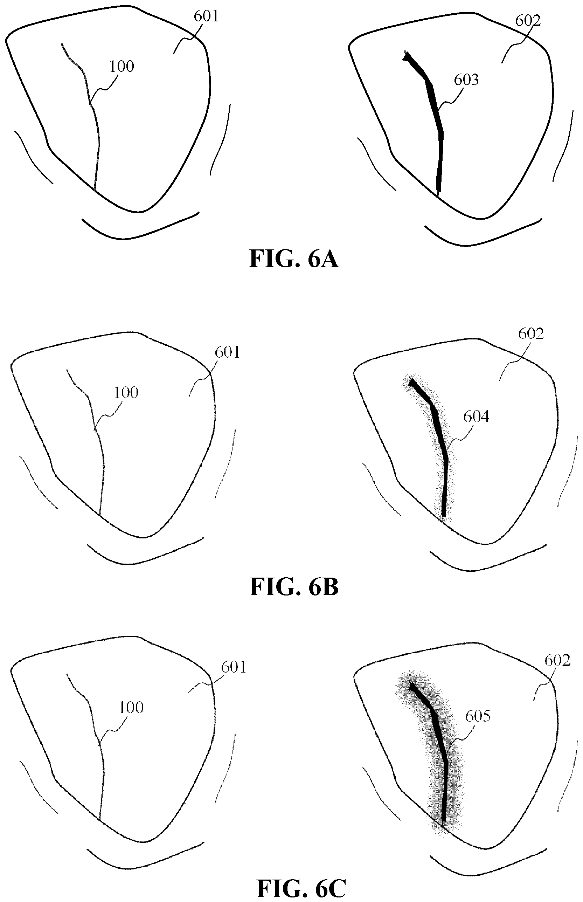

FIGS. 6A-6C illustrate three severity level values of the detected tooth crack 100, where the severity level values are detectable on the digital 3D model using the fluorescence information. The tooth crack 100 of FIG. 6A may have low severity value (low risk), the tooth crack 100 of FIG. 6B may have medium severity value (medium risk), while the tooth crack 100 of FIG. 6C may have a high severity value (high risk).

Tooth 601 of FIG. 6A is displayed with its corresponding color information. Tooth 602 represents the same tooth displayed with the corresponding fluorescence information. By visualizing the tooth 102 with the fluorescence information, the severity level of the tooth crack is made assessable. This can be observed by comparing the same tooth in different imaging modalities, such as comparing the tooth 601 in natural color with the same tooth 602 with fluorescence texture applied.

Similar applies to FIGS. 6B and 6C. Tooth 601 of FIG. 6B is displayed with the corresponding color information. Tooth 602 of FIG. 6B represents the same tooth as tooth 601 of FIG. 6B, displayed with the corresponding fluorescence information. Tooth 601 of FIG. 6C is displayed with the corresponding color information. Tooth 602 of FIG. 6C represents the same tooth as tooth 601 of FIG. 6C, displayed with the corresponding fluorescence information.

While the tooth crack 100 may be observable in the tooth 601 displayed with color information in FIGS. 6A-6C, the severity levels of the tooth cracks 100 may be difficult to evaluate based solely on the color information. For that reason the fluorescence information may further be considered.

FIG. 6A illustrates a low risk tooth crack 603. This severity level may be determined based on the fluorescence information associated with the tooth crack 603. This may be concluded by observing a color change associated with the fluorescence information of the tooth crack 603 of the tooth 602. In this case no color change along the tooth crack 603 is observed and the tooth crack 603 may therefore be classified as low risk. In this case no treatment besides monitoring of the tooth crack 603 may be needed.

FIG. 6B illustrates a medium risk tooth crack 604. Tooth 601 and the tooth 602 represent the same tooth of the same dental situation, acquired with different scanning modalities of the intraoral scanner 801. The tooth crack 100 is thus the same as the tooth crack 604 in FIG. 6B. A same tooth may be displayed as tooth 601 with the associated color information and as the tooth 602 with the associated fluorescence information. Based on the fluorescence information it may be determined that the tooth crack 604 is of medium risk. This may be concluded by observing the color change associated with the fluorescence information of the tooth crack 604 of the tooth 602. In this case observed change in red color along the tooth crack 604 may be higher than a first threshold and the tooth crack 604 may be classified as medium risk. A treatment recommendation may subsequently be generated. For example, a non-invasive treatment for the tooth crack 604 may be automatically suggested.

FIG. 6C illustrates a high risk tooth crack 605. Tooth 601 and the tooth 602 in FIG. 6C represent the same tooth of the same dental situation, acquired with different scanning modalities of the intraoral scanner 801. Thus, in FIG. 6C, the tooth crack 100 is the same as the tooth crack 605. The tooth 601 may be displayed with the associated color information while the tooth 602 may be displayed with the associated fluorescence information. Based on the fluorescence information it may be determined that the tooth crack 605 is of high risk. This may be concluded by observing the color change associated with the fluorescence information of the tooth crack 605 of the tooth 602. In this case observed red color change along the tooth crack 605 may be higher than a second threshold and the tooth crack 605 is classified as high risk. In this case a treatment for the tooth crack 605 may be automatically recommended. The second threshold may be greater than the first threshold. These thresholds may be empirically derived.

The severity of the tooth crack 100 may be, in an example, the depth of the tooth crack 100.

In the context of FIGS. 6A-6C, the fluorescence information may be used to post-process the output 305, 405 of the trained neural network 300, 400 to determine the severity level of the detected tooth crack 100.

Alternatively, the input 301, 401 of the trained neural network 300, 400 may comprise the fluorescence information, and the trained neural network 300, 400 may provide, as part of the output 305, 405 also the severity value of the detected tooth crack 100.

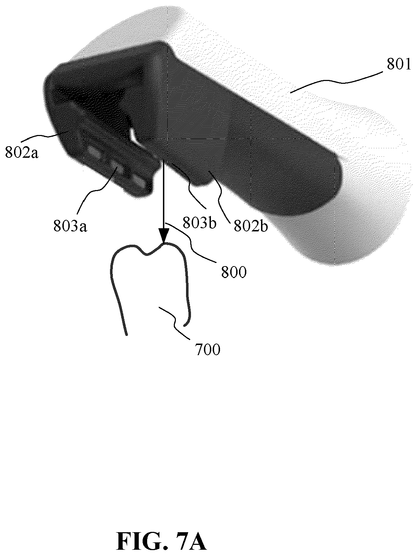

FIGS. 7A-7C illustrate detection of the tooth crack using infrared image data. Methods to detect tooth cracks with an intraoral scanner using near-infrared imaging rely on the fact that the tooth enamel material is highly transparent to near-infrared light, and because of that, the tooth cracks can be identified on the images recorded by the intraoral scanner using near-infrared light. Generally, techniques such as near-infrared (NIR) reflection and near-infrared (NIR) transillumination may be used to detect tooth cracks.

Teeth scanning using near-infrared (NIR) transillumination may be used directly to detect tooth cracks due to high transparency of the enamel. Scanning using the NIR transillumination is also relatively fast, making it possible to image crack formation in real time. However, types of cracks may not be possible to distinguish, nor the accurate crack depth information. Furthermore, likelihood of a misdiagnose exists as non-structural tooth cracks may appear as structural tooth cracks.

By including the data obtained by near-infrared transillumination and/or near-infrared reflection to the input 301, 401 of the trained neural network 300, 400, detection of tooth cracks may be improved.

Shown in FIG. 7A is the intraoral scanner 801, more specifically its part referred to as a tip or a head, configured to irradiate the tooth 700 with near-infrared light and record the irradiated near-infrared light in its image sensor located in a body of the intraoral scanner 801.

The intraoral scanner 801 may comprise arms or holders 802a, 802b to which a first and a second near-infrared light sources 803a, 803b may be attached (light source 803b is located on the inside of the arm 802b and thus not clearly visible in FIG. 7A). The tip of the intraoral scanner 801 comprising the arms 802a, 802b may be removably attached to the body of the scanner 801. In this configuration, the intraoral scanner 801 may be configured to directly irradiate the tooth 700 from its buccal and/or lingual surface when the tip of the intraoral scanner 801 is positioned parallel to the occlusal surface of the tooth 700 such that the arms 802a, 802b are in contact with the buccal and the lingual surface of the tooth 700 (illustrated with arrow 800).

The near-infrared images obtained by the intraoral scanner 801 in this configuration may be aligned to the corresponding digital 3D model. In this way, the exact position where each near-infrared image is taken may be registered onto the digital 3D model.

The first and the second near-infrared light sources 803a, 803b may be configured to irradiate the buccal and/or lingual surface of the tooth 700 with near-infrared light in mutually opposing directions.

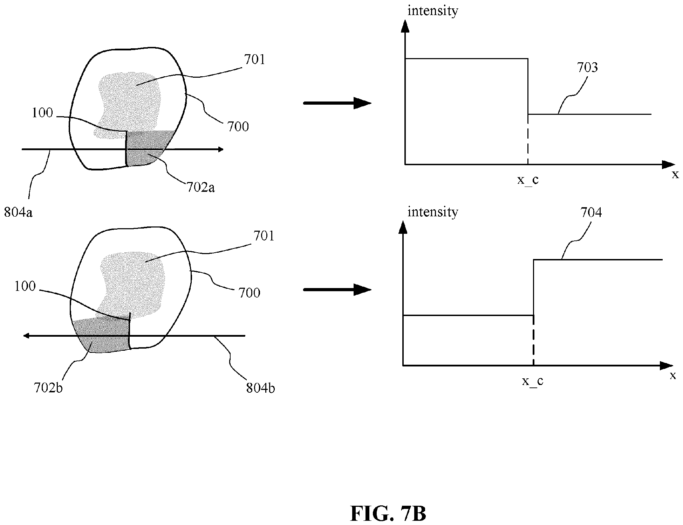

FIG. 7B illustrates views of the tooth 700 from its occlusal surface, as seen by the intraoral scanner 801 during alternate activation of the near-infrared light sources 803a, 803b of the arms 802a, 802b. In this case, an infrared light absorbing dye (disclosing agent) is infiltrated into the tooth crack 100.

Top part of FIG. 7B illustrates a first direction 804a of the near-infrared light emitted from the first near-infrared light source 803a while the second near-infrared light source 803b is inactive. This may result in generating a first near-infrared image 705. As the light passes through the tooth crack 100, a shadow 702a is created due to absorption of the light, by the absorbing dye, in the tooth crack 100. The tooth crack 100 may thus be identified as the region of the tooth 700 from where the shadow 702a appears, namely as a border between a bright region and the shadow 702a. It may be observed that the contrast between the bright region of enamel and the dark region of enamel (shadow 702a) is higher than the observable contrast in the dentin 701 due to the tooth crack 100. This is due to the fact that dentine is less transparent to the near-infrared light than the enamel.

A plot 703 in the top part of FIG. 7B may be referred to as an intensity plot 703 over distance, and represents intensity values of the first near-infrared image 705 along the beam of the near-infrared light in the first direction 804a. A drop in intensity may be observed at a position x_c of the tooth crack 100.

Bottom part of FIG. 7B illustrates a second direction 804b of the near-infrared light emitted from the second near-infrared light source 803b while the first near-infrared light source 803a is inactive. In this way a second near-infrared image 706 is generated, depicting substantially the same dental object as the first near-infrared image 705, meaning that the scanner 801 does not move significantly with respect to the teeth. As the light passes through the tooth crack 100, a shadow 702b is created due to absorption of the light, by the absorbing dye, in the tooth crack 100. The tooth crack 100 may thus be identified as the region of the tooth 700 from where the shadow 702b appears. The tooth crack 100 may be identified as a transitional region (or a border region) between a bright area and the shadow 702a, 702b of the tooth 700, wherein the transitional region is observable along the direction of emitted near-infrared light.

The plot 704 in the bottom part of FIG. 7B may be referred to as the intensity plot 704 over distance, and represents intensity values of the second near-infrared image 706 along the beam of the near-infrared light in the second direction 804b. An increase in intensity may be observed at the position x_c of the tooth crack 100.

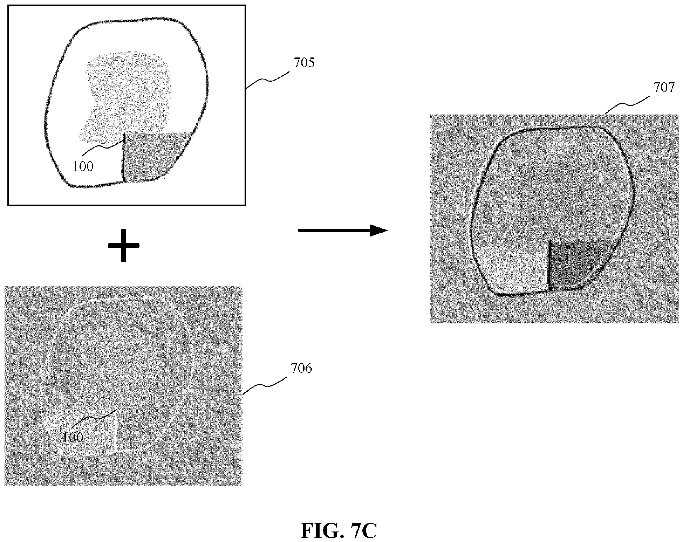

The obtained first near-infrared image 705 and the second near-infrared image 706 may be combined in a way that the contrast of the images is improved, thus improving the detectability of the tooth crack 100. An example of such combination is illustrated in FIG. 7C where the two near-infrared images 705, 706 are subtracted from each other to generate a modified near-infrared image 707 for the purpose of enhancing contrast and thereby improving the accuracy of tooth crack 100 detection. The subtraction may be on pixel-by-pixel level. The accuracy of the tooth crack 100 detection on the modified near-infrared image 707 is thereby improved compared to the tooth crack 100 detection on individual images 705, 706.

The method for identifying the tooth crack 100 using near-infrared image data may comprise:
- receiving the first near-infrared image 705 depicting the tooth 700 illuminated only with the first near-infrared light source 803a through a first surface of the tooth 700,
- receiving the second near-infrared image 706 depicting the tooth 700 illuminated only with the second near-infrared light source 803a through a second surface of the tooth 700, wherein the first surface and the second surface are different approximal tooth surfaces,

- generating the modified near-infrared image 707 by subtracting the second near-infrared image 706 from the first near-infrared image 705 or by subtracting the first near-infrared image 705 from the second near-infrared image 706,
- detecting the tooth crack 100 based on analysis of the modified near-infrared image 707.

The analysis of the modified near-infrared image 707 may be an image analysis algorithm to detect contours, contrast and/or colors. The analysis of the modified near-infrared image 707 may alternatively or additionally be based on the trained neural network 300, 400 for tooth crack detection. The modified near-infrared image 707 may, in this case, be input into the trained neural network as part of the digital 3D model.

Approximal tooth surfaces may be understood as a facial (labial or buccal) surface and a lingual surface of the tooth.

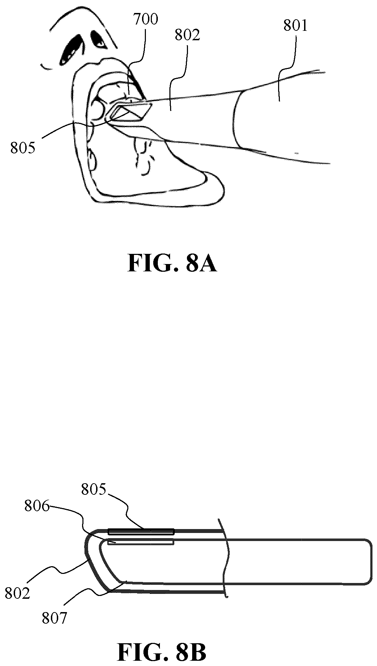

FIG. 8A illustrates detection of the tooth crack 100 in the scan data obtained while biting on the intraoral scanner 801. Scan data may be obtained of the patient's tooth 700 suffering from the tooth crack 100 while the patient is biting on a tip 802 of the intraoral scanner 801. Changes associated with the tooth crack 100, due to biting action, may be recorded. The changes may then be displayed to the user.

The changes to the tooth crack 100 due to biting action may be recognized in the 2D image data captured by the intraoral scanner 801 or may be recognized in the process of building the digital 3D model. The changes may be recognized automatically.

The recorded changes may relate to the severity level of the tooth crack 100. In one example a digital image correlation (DIC) can be used to detect tooth cracks. In this method the 2D image data, captured by the intraoral scanner 801 during the biting, may be used to compute a strain field. The strain field may then be used to determine a location of the tooth crack 100 and/or the depth of the tooth crack 100.

To see the tooth crack 100 open and/or close, the patient may bite on a window 805 of the tip 802. The window 805 may be made of sapphire glass as well as other glass types for which the hardness is above the hardness of enamel. A further example of the material suitable for the window 805 is borosilicate glass.

In an example, the window 805 may be a prism of thickness in range of 1.5 millimeters to 30 millimeters such as to provide sufficient resistance to forces from biting action.

FIG. 8B shows the tip 802 of the intraoral scanner 801 that is intended for a single use (a single-use tip). The window 805 of the tip 802 and an inner window 806 of a core 807 of the tip may mutually overlap. The two windows 805, 806 may be made from the same material. The inner window 806 may be a prism.

Optimally, the material used for the window 805 and/or the inner window 806 may have hardness higher than enamel hardness. Optionally, the material may be of hardness lower than enamel hardness, especially if the tip 802 is exchanged frequently, such as after 10-100 bites. The material with hardness lower than the enamel hardness may have Vicker Hardness Number (VHN) hardness value of approximately half of the enamel hardness value, for example value of around 135.

To increase the strength of the tip 802, a gap between the window 805 and the inner window 806 may be minimized. In an example, the window 805 and the inner window 806 may be in contact. This may enable reducing of forces in the window 805 by allowing the inner window to take some of the load caused by the patient's bite. The tip 802 may be configured such that the window 805 and the inner window 806 are in contact in a loaded scenario when the patient is biting on the tip 802, and that the window 805 and the inner window 806 are separated with the gap in an unloaded scenario when the patient is not biting on the tip 802.

In one example, recording changes associated with the tooth crack 100 in the patient's tooth 700 may comprise recording pressure exerted by the tooth 700 suffering from the tooth crack 100 while the patient is biting on the tip 802 of the intraoral scanner.

The tip of the intraoral scanner 802 may be adapted to this purpose, for example by comprising an outer metallic frame. The frame could be made of a polymer material with a high compressive yield strength such as polyarylamide, polyimide (PI), polyamide-imide, polyimide liquid crystal polymer or poly. Alternatively, ceramics such as zirconia may be used.

The tip 802 of the intraoral scanner may comprise a pressure sensor, for example in the outer metallic frame. The pressure sensor may be a strain gauge. The pressure sensor may alternatively be comprised in the inner window 806. Alternatively, the pressure sensor may be made up from several components in combination such as optical measuring equipment combined with a spring structure in the window 805 or the frame

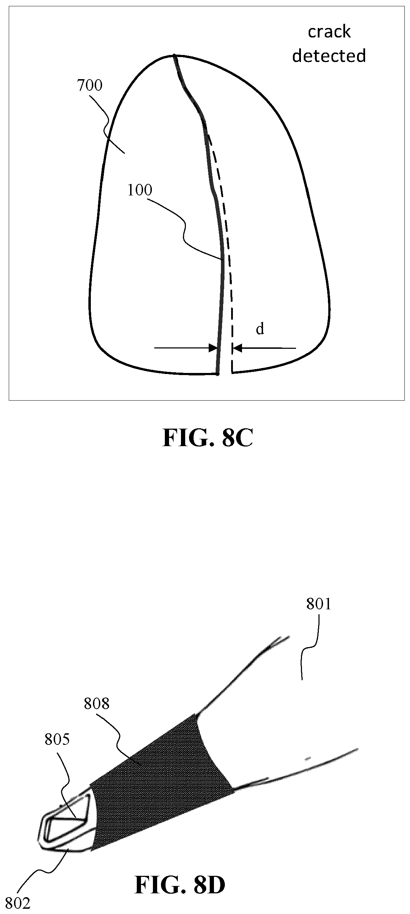

FIG. 8C shows the changes associated with the tooth crack 100, such as the tooth crack 100 opening up, due to the biting action. For example, a distance d may represent a maximum amount by which the tooth crack 100 opens up. Changes associated with the tooth crack 100, as shown in FIG. 8C may be displayed on the display of the computer 1110.

This information, such as opening up of the tooth crack 100 may be captured in the 2D image data by the intraoral scanner 801.

A dental scanning system 1100 according to an embodiment may comprise the intraoral scanner 801 adapted for recording changes in the patient's dentition while the patient bites on the tip 802 of the intraoral scanner. The changes may relate to detecting the tooth crack 100 and/or detecting changes in severity of the tooth crack 100.

FIG. 8D illustrates another example of the intraoral scanner 801 adapted for biting by the patient in order to detect tooth cracks.

In FIG. 8D a sleeve 808 is shown fitted over the tip 802. The sleeve 808 may be made of a material with sufficient hardness that will allow isolating the pain experience to be in the tooth crack and not on the surface of the teeth.

The examples of materials suitable for the sleeve 808 may be polysulfone (PSU) plastic material in case of a reusable tip or Poly propylene (ABS) for the single-use tip.

To protect the reusable tip, the sleeve 808 may be used. The sleeve 808 may be made out of a rubber-like materials such as: silicone, thermoplastic elastomers (TPE), thermoplastic polyurethane (TPU), polypropylene (PP), polyethylene (PE) or polyamide (PA).

Bite marks that may occur on the single-use tip would not represent an issue as such tip would be discarded after use.

In some cases where the tip 802 may come under significant forces from the biting. The tip 802 may in such cases comprise additional reinforcement, for example in form of a thicker aluminum casing, for example greater than 0.3 millimeters. Stainless steel such as AISI 316 or ceramic materials could alternatively be used.

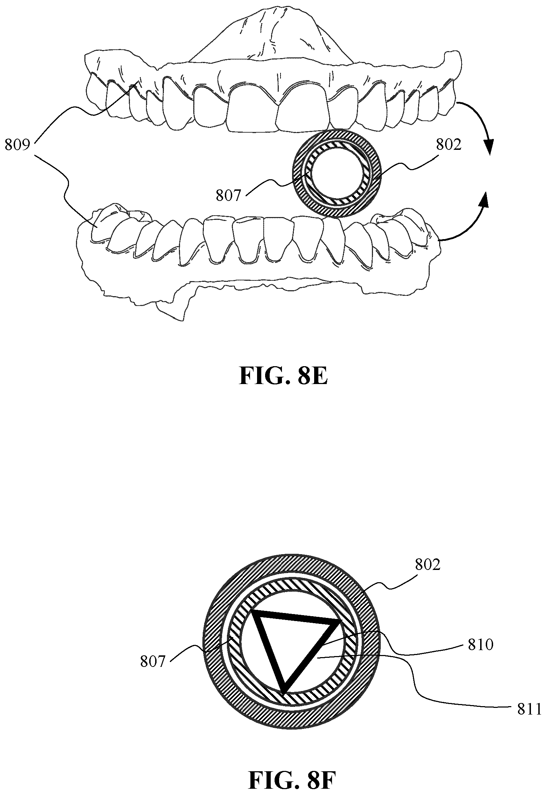

FIG. 8E illustrates the patient's dental situation 809 in a bite arrangement with the intraoral scanner 801 (a cross section of the scanner tip 802 is shown). In the illustration the patient bites on the tip 802 of the intraoral scanner 801 with anterior teeth. The tip 802 may be the single-use tip. The core 807 of the tip may be configured to handle additional forces due to the bite arrangement. The core 807 may comprise an aluminum casing, for example with a thickness greater than 0.3 millimeters. This minimum level of thickness may be sufficient for the core 807 to withstand patient's biting forces without further structural reinforcement. Stainless steel such as AISI 316 or ceramic materials could alternatively be used.

Alternatively, the core 807 may be of a thickness less than 0.3 millimeters and may comprise an internal reinforcement structure 810, as shown in FIG. 8F. The reinforcement structure 810 may provide required strengthening of the core 807 in case the thickness of the core 807 is less than 0.3 millimeters. The reinforcement structure 810 may be in form of a web of stiffeners, for example of a triangular profile as in FIG. 8F, designed to improve the rigidity and resistance to bite force of the scanner tip 802. The reinforcement structure 810 may comprise an opening 811 for allowing electronic wiring and light rays to pass through.

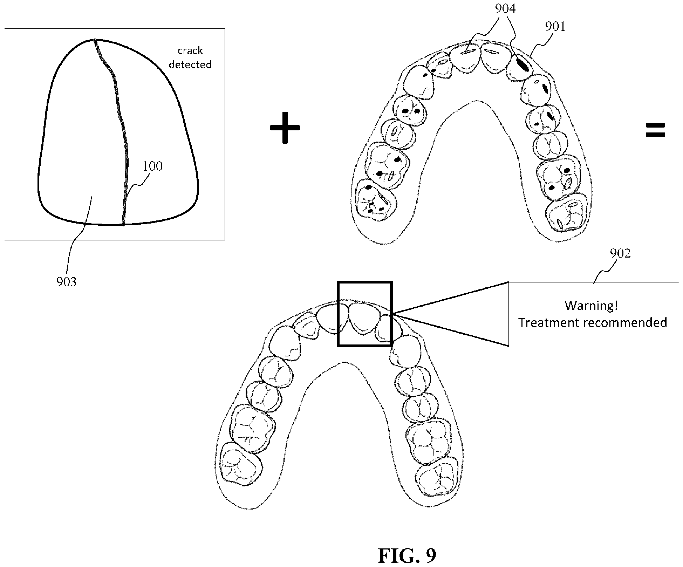

FIG. 9 illustrates detecting a tooth crack characteristic based on a digital measurement of the patient's biting force 901.

The digital measurement of the patient's biting force 901 may be obtained by recording the patient's bite on a device equipped with a suitable sensor. It may visualize parts of the teeth where patient's jaw come in contact, as well as to provide indication about force exerted in those parts of the teeth. The parts of the teeth where patient's jaw come in contact are illustrated as surfaces 904 while different colors of the surfaces 904 indicate contact force when jaw is closed. Alternatively, a digital measurement comprising only the parts of the teeth where patient's jaw come in contact may be used.

This digital measurement 901 may be received, for example by the processor of the dental scanning system 1100, and evaluated in combination with the indication of the detected tooth crack 100.

In an example, it may be determined if the detected tooth crack 100, thereby the corresponding tooth 903, is in contact with an antagonist tooth based on the received digital measurement of the patient's biting force 901. The antagonist tooth is a tooth of opposite jaw that comes in contact with the tooth 903 under evaluation. A warning 902 may be generated if the determination is positive.

Additionally or alternatively, if the tooth 903 and its antagonist tooth do come in contact, a corresponding force of that contact may be determined. Based on the force, a further warning may be generated. For example, if the force is above a predetermined threshold, the warning may be generate and a recommendation for a treatment may be provided.

In another embodiment, not shown in FIG. 9, the method may comprise receiving a digital measurement of the patient's bite showing distances between teeth of the patient's jaws and contact points of the teeth of the patient's jaws. This digital measurement of the patient's bite may be different from the digital measurement of the patient's biting force 901.

Similarly, a determination whether the detected tooth crack 100, or the associated tooth 903, is in contact with the antagonist tooth may be performed. Further, a similar warning, for example recommending a treatment, may be generated if the determination is positive.

Obtained information regarding the contact with the antagonist tooth may be recorded in a dental record of the patient such as a dental chart.

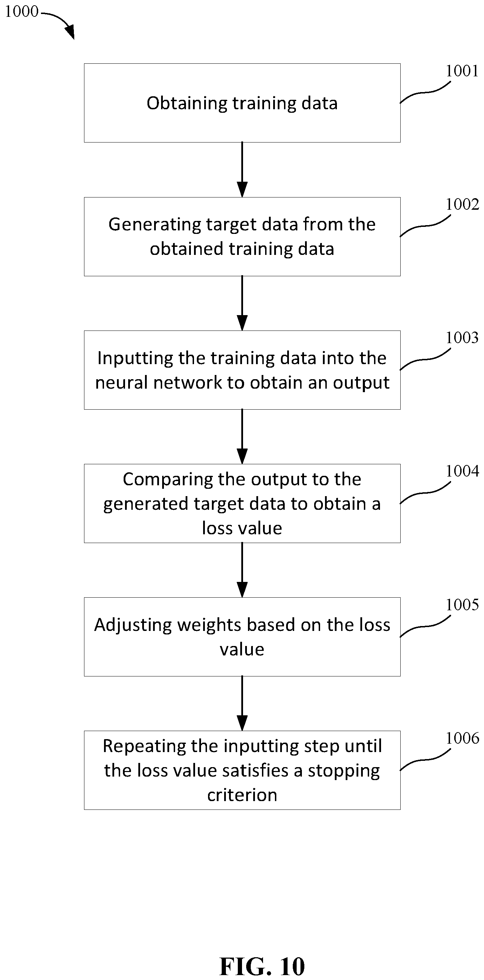

FIG. 10 is a flowchart illustrating a method 1000 for training a neural network for detecting tooth cracks 100.

Step 1001 of the method 1000 illustrates obtaining a training data for the neural network, wherein the training data comprises a first training sample (a digital 3D model) with a tooth crack disclosed and a corresponding second training sample without the tooth crack disclosed.

The training data may comprise a plurality of different training samples. This data may be a plurality of intraoral 3D scans obtained by scanning various patients. For example, the training data may comprise the first training sample of a dental situation with a tooth crack disclosed. The disclosed tooth crack may serve as ground truth. Crack presence may be determined for example by using the common disclosing agent. Next, the second training sample of the same dental situation without the tooth crack disclosed may be obtained, for example when the disclosing agent is removed.

Step 1002 illustrates generating target data, or "ground truth", by identifying the tooth crack disclosed on the first training sample and transferring the identified tooth crack onto the second training sample.

Step 1003 of the method 1000 illustrates inputting the second training sample of the dental situation into the neural network to obtain an output from the neural network.

Step 1004 illustrates comparing the output from the neural network to the generated target data to obtain a loss.

Step 1005 illustrates adjusting weights of the neural network based on the comparing step. The obtained loss should be minimized, for example by using stochastic gradient descent algorithm (SGD).

Step 1006 illustrates repeating the inputting step until the loss satisfies a stopping criterion. The training process is therefore an iterative process. The training process may be stopped when the loss reaches a stopping criterion. For example, the stopping criterion may be a threshold value for the loss. The stopping criterion may also be a number of epochs (training cycles) after which performance metrics cease to increase.

The particular advantage of the mentioned training method is that target data may be generated without human annotation of tooth cracks.

Identifying the tooth crack disclosed on the first training digital 3D model may comprise:
- comparing a color of a facet of the first training digital 3D model to a reference color representing the disclosing agent to compute an Euclidian distance between the color of the facet and the reference color. Further, the facet may be assigned to a disclosing agent mask if the Euclidian distance is lower than a color distance threshold.

The trained neural network 300, 400 may be trained, as discussed above, based on the training data annotated automatically. Alternatively, the training samples comprising tooth cracks serving as the ground truth may be manually annotated. The training data may comprise several hundred or several thousands of training samples. The training data may preferably comprise more than 500 training samples.

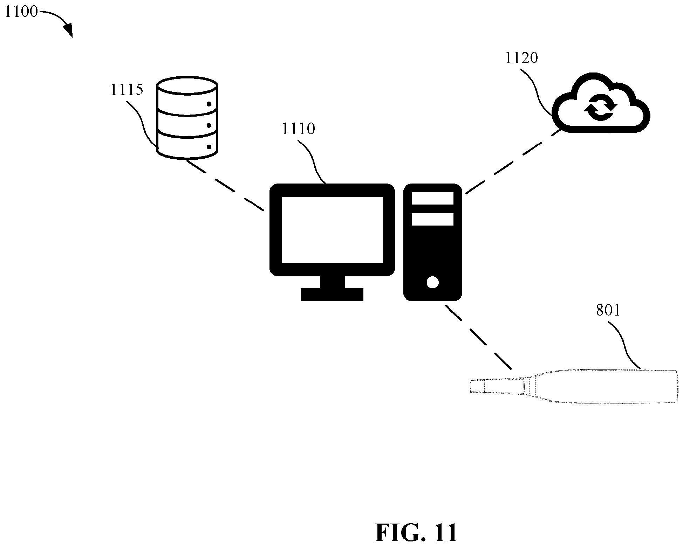

FIG. 11 illustrates a dental scanning system 1100 according to the disclosure.

The dental scanning system 1100 which may comprise a computer 1110 capable of carrying out any method of the disclosure. The computer may comprise a wired or a wireless interface to a server 1115, a cloud server 1120 and the intraoral scanner 801. The intraoral scanner 801 may be capable of recording the scan data comprising geometrical information, natural color information, infrared information and/or fluorescence information associated with the patient's dentition. The intraoral scanner 801 may be equipped with various modules such as a fluorescence module and/or an infrared module and thereby capable of capturing information relevant for diagnosing dental conditions such as caries, tooth cracks, gingivitis and/or plaque.

The dental scanning system 1100 may comprise a data processing device configured to carry out the method according to one or more embodiments of the disclosure. The data processing device may be a part of the computer 1110, the server 1115 or the cloud server 1120.

A non-transitory computer-readable storage medium may be comprised in the dental scanning system 1100. The non-transitory computer-readable medium can carry instructions which, when executed by a computer, cause the computer to carry out the method according to one or more embodiments of the disclosure.

A computer program product may be embodied in the non-transitory computer-readable storage medium. The computer program product may comprise instructions which, when executed by a computer, cause the computer to perform the method according to any of the embodiments presented herein.

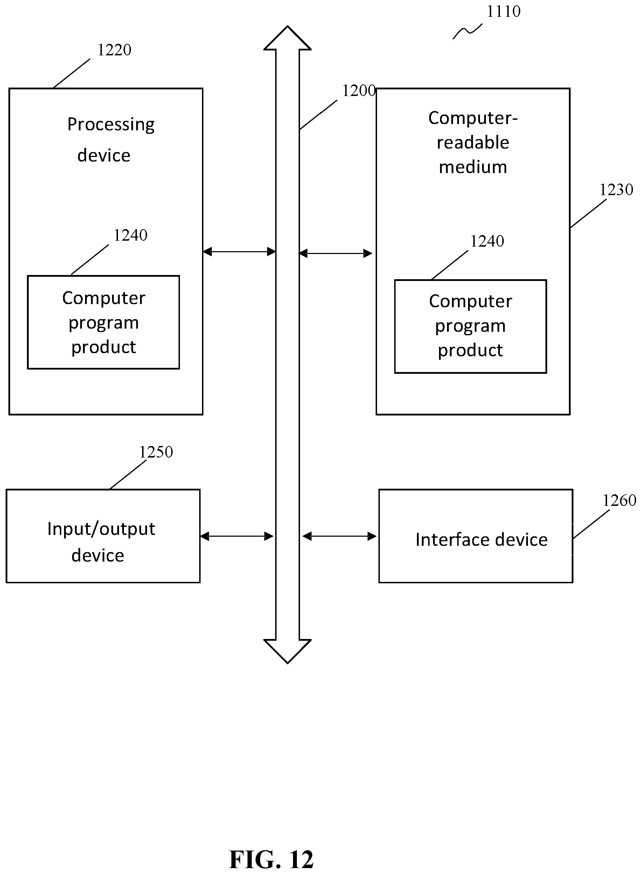

FIG. 12 illustrates an example of a computer architecture of a computer 1110 capable of carrying out a method according to the disclosure.

Various components of the computer 1110 may communicate via a bus 1200.

The computer 1110 may comprise the data processing device 1220 (referred to also as a processor or a processing device). The data processing device 1220 may be any central processing unit (CPU), microprocessor, microcontroller, computational or programmable device or circuit configured for executing instructions to carry out the method of any one or more of the presented embodiments.

The computer program product 1240, comprising the instructions to carry out the method of any one or more of the presented embodiments, may be stored on the data processing device 1220. Alternatively or additionally, the computer program product 1240, comprising the instructions to carry out the method of any one or more of the presented embodiments, may be stored on a computer-readable medium 1230, more specifically on a non-transitory computer-readable medium 1230. Examples of the computer-readable medium 1230 include magnetic storage media such as a magnetic disk or magnetic tape, optical storage media such as an optical disc, optical tape, machine readable bar code, solid state electronic storage devices such as random access memory (RAM), read only memory (ROM), or any other physical device or medium configured to store the computer program product 1240.

The computer 1110 may further comprise an input/output device 1250 such as a keyboard, a touchscreen, a microphone, a mouse, a display, a graphical user interface (GUI), a loudspeaker etc.

The computer 1110 may be connected to the server 1115, the cloud 1120 and/or the intraoral scanner 801 via an interface device 1260 which may be a wired and/or wireless communication interface device including Wi-Fi, Bluetooth, LAN, etc.

References throughout this disclosure to certain features, advantages, or similar does not imply that all of the features and advantages of the present disclosure should be or are in any single embodiment. Instead, it is to be understood that a specific feature, or characteristic described in connection with an embodiment is included in at least one embodiment of the disclosure. Thus, discussion of the features and advantages, and similar language, throughout this disclosure may, but do not necessarily, refer to the same embodiment.

## Claims

1. A computer-implemented method for detecting tooth cracks on a digital 3D model of a dental situation, the method comprising:
- receiving the digital 3D model of the dental situation;
- generating an input for a trained neural network for tooth crack detection, wherein the input comprises at least a part of the digital 3D model with associated color information;
- detecting a tooth crack on the at least part of the digital 3D model by applying the trained neural network to the generated input;
- determining a severity level of the detected tooth crack based on fluorescence information associated with the at least part of the digital 3D model; and
- displaying the at least part of the digital 3D model, the detected tooth crack and the severity level of the detected tooth crack.

2. The method according to claim 1, wherein detecting the tooth crack comprises detecting a position of the tooth crack on the at least part of the digital 3D model and/or a degree of confidence representing a probability that the tooth crack is detected correctly.

3. The method according to claim 1 or 2, wherein detecting the tooth crack comprises detecting a size of the tooth crack.

4. The method according to any previous claim, wherein determining the severity level of the detected tooth crack comprises computing a bacteria presence score indicating an amount of bacteria present in the detected tooth crack.

5. The method according to the previous claim 4, wherein the bacteria presence score is computed as a function of a red (R) fluorescence value and/or a green (G) fluorescence value of the fluorescence information.

6. The method according to any previous claim, wherein the input further comprises a modified near-infrared image, wherein the modified near-infrared image is obtained by mutually subtracting a first near-infrared image and a second near-infrared image, wherein the first near-infrared image depicts a tooth illuminated by a near-infrared light source only through a first surface, wherein the second near-infrared image depicts the tooth illuminated by a near-infrared light source only through a second surface, and wherein the first surface and the second surface are different approximal surfaces of the tooth.

7. The method according to any previous claim, wherein the input further comprises combined image data associated with the at least part of the digital 3D model, wherein the combined image data is generated as a function of infrared image data, the fluorescence information and/or the color information.

8. The method according to any previous claim, further comprising receiving a digital measurement of a patient's bite, wherein the digital measurement of the patient's bite comprises distances between teeth of patient's jaws and contact points of the teeth of the patient's jaws.

9. The method according to the previous claim 8, further comprising determining that the detected tooth crack is in contact with an antagonist tooth based on the received digital measurement of the patient's bite.

10. The method according to any previous claim, wherein detecting the tooth crack comprises determining by the trained neural network, a curve representing the tooth crack.

11. The method according to the previous claim 10, further comprising computing the length of the curve, such that the length of the curve represents the length of the detected tooth crack.

12. The method according to any previous claim, wherein detecting the tooth crack comprises determining a number of facets of the at least part of the digital 3D model representing the tooth crack.

13. The method according to the previous claim 12, further comprising computing a line length within the determined number of facets such that the computed line length represents the length of the detected tooth crack.

14. The method according to any previous claim, further comprising receiving a digital measurement of the patient's bite force and determining that the detected tooth crack is in contact with the antagonist tooth based on the received digital measurement of the patient's bite force.

15. The method according to the previous claim 14, further comprising displaying the detected tooth crack in contact with the antagonist tooth on the digital 3D model.
